# EUROPEAN PATENT APPLICATION

(11) **EP 4 257 959 A1**
(43) Date of publication of application: **11.10.2023**
(21) Application number: 23163534.3
(22) Date of filing: 22.03.2023
(51) Int. Cl.: G01N 21/84, G01N 21/88, G01N 21/91, G01N 21/95, G01R 31/34, G01R 31/62, G01R 31/72, G01N 21/64

(54) **METHOD OF QUALITY CONTROL AND SERVICE INSPECTION FOR METALLIC ELECTRO-MECHANICAL COMPONENTS**

(30) Priority: 05.04.2022 US 202217713903
(71) Applicant: Goodrich Corporation, Charlotte, NC 28217-4578 (US)
(72) Inventor: GURVICH, Mark R., Middletown, CT 06457 (US); ZAFIRIS, Georgios S., Glastonbury, CT 06033 (US); SYPEK, John T., Bolton, CT 01043 (US)
(74) Representative: Dehns

(57) **Abstract**

The present disclosure provides assemblies, systems and methods of quality control and service inspection for metallic electro-mechanical components. More particularly, the present disclosure provides assemblies, systems and methods of quality control and service inspection for metallic electro-mechanical components (e.g., stator components) utilizing fluorescent agents. The present disclosure provides both (i) surface/assembly designs, and (ii) methods of their inspection. In the present disclosure, fluorescent agents can be mixed with protective and/or added layers to serve as bright contrast media under a light source (e.g., ultraviolet ("UV") light source). It is noted that in a case of imperfect coating or service corrosion, gaps in reflection can serve as clear indications of either inadequate quality or excessive corrosion-driven degradation of the components.

## Description

### TECHNICAL FIELD

The present disclosure relates to assemblies, systems and methods of quality control and service inspection for metallic electro-mechanical components and, more particularly, to assemblies, systems and methods of quality control and service inspection for metallic electro-mechanical components (e.g., stator components) utilizing fluorescent agents.

### BACKGROUND

In general, to protect electro-mechanical components from corrosion, a protective coating can be used as an applied sprayed barrier layer (e.g., paint). However, there is a risk of inadequate coating coverage (e.g., with spottiness of the applied layer). Therefore, quality control of coating coverage can be a challenge for various reasons (e.g., due to complex surface shapes, hard-to-see internal corners, small sizes of imperfectly coated areas (spottiness), etc.).

Another challenge is expected corrosion during operation due to harsh environments or the like. Thus, detection of operational corrosion can be needed for pro-active maintenance (e.g., replacement, repair, etc.) and/or optimization of a protective coating. However, accurate detection of operational corrosion can be a difficult task (e.g., due to poor contrast, limited visibility and access, and potential subjectivity of assessment, especially, at the beginning of a corrosion process).

### BRIEF DESCRIPTION

The present disclosure provides assemblies for quality control and service inspection for metallic electro-mechanical components, and improved systems/methods of quality control and service inspection for metallic electro-mechanical components. More particularly, the present disclosure provides embodiments that may result in improved assemblies, systems and methods of quality control and service inspection for metallic electro-mechanical components (e.g., aircraft stator components) utilizing fluorescent agents.

The present disclosure provides for a coated assembly including a metallic electro-mechanical component having an outer surface; and a protective coating or a first added layer positioned proximal to the outer surface, the protective coating or the first added layer comprising a first fluorescent agent; and wherein the first fluorescent agent operates as bright contrast media under a light source to identify imperfect coating or coating degradation during service of the metallic electro-mechanical component.

In embodiments, the protective coating is positioned on the outer surface, and the first added layer is positioned on the protective coating.

In embodiments, the first added layer comprises the first fluorescent agent, and the protective coating is free of the first fluorescent agent.

In embodiments, the protective coating is positioned on the outer surface, and the metallic electro-mechanical component is free of the first added layer, the protective coating comprising the first fluorescent agent.

In embodiments, the protective coating comprises the first fluorescent agent, and the first added layer comprises a second fluorescent agent.

In embodiments, the first added layer is positioned on the outer surface, and the protective coating is positioned on the first added layer, the first added layer comprising the first fluorescent agent.

In embodiments, the protective coating comprises a second fluorescent agent.

In embodiments, a second added layer is positioned on the protective coating, the second added layer comprising a second fluorescent agent.

In embodiments, the protective coating comprises a third fluorescent agent.

In embodiments, the metallic electro-mechanical component is a stator metallic component, and the first fluorescent agent is a dye or a pigment; and the outer surface defines a plurality of protrusions and recesses.

The present disclosure provides for an inspection method including providing a metallic electro-mechanical component having an outer surface; and positioning a protective coating or a first added layer proximal to the outer surface, the protective coating or the first added layer comprising a first fluorescent agent; and applying a light source onto the metallic electro-mechanical component, with the first fluorescent agent operating as bright contrast media under the light source to identify imperfect coating or coating degradation during service of the metallic electro-mechanical component.

In embodiments, the protective coating is positioned on the outer surface, and the first added layer is positioned on the protective coating; and the first added layer comprises the first fluorescent agent, and the protective coating is free of the first fluorescent agent; and wherein by applying light via the light source to the component, areas of imperfect or degraded coating of the component show an indication of no reflection of light, in contrast with neighboring undamaged regions of the component having bright reflection from the first fluorescent agent, and the areas of no reflection indicate the location and size of imperfect or degraded coating to the component during service inspection.

In embodiments, the protective coating is positioned on the outer surface, and the metallic electro-mechanical component is free of the first added layer, the protective coating comprising the first fluorescent agent; and wherein by applying light via the light source to the component, areas of imperfect or degraded coating of the component show an indication of no reflection of light, in contrast with neighboring undamaged regions of the component having bright reflection from the first fluorescent agent, and the areas of no reflection indicate the location and size of imperfect or degraded coating to the component during service inspection.

In embodiments, the protective coating is positioned on the outer surface, and the first added layer is positioned on the protective coating; and the protective coating comprises the first fluorescent agent, and the first added layer comprises a second fluorescent agent; and wherein by applying light via the light source to the component, areas of imperfect or degraded coating of the component show an indication of no reflection of light, in contrast with neighboring undamaged regions of the component having bright reflection from the first or second fluorescent agents, and the areas of no reflection indicate the location and size of imperfect or degraded coating to the component during service inspection.

In embodiments, the first added layer is positioned on the outer surface, and the protective coating is positioned on the first added layer, the first added layer comprising the first fluorescent agent; and wherein by applying light via the light source to the component, areas of spottiness of the protective coating show an indication of reflection from the first fluorescent agent of the first added layer, in contrast with neighboring protective coated regions of the component with no reflection from the first fluorescent agent, and the areas of reflection indicate the location and size of imperfections of the protective coating of the component during a quality control assessment.

In embodiments, the first added layer is positioned on the outer surface, and the protective coating is positioned on the first added layer, the first added layer comprising the first fluorescent agent; and further comprising a second added layer positioned on the protective coating, the second added layer comprising a second fluorescent agent; and wherein by applying light via the light source to the component, areas of imperfect or degraded coating of the component show an indication of no reflection of light, in contrast with neighboring undamaged regions of the component having bright reflection from the first or second fluorescent agents, and the areas of no reflection indicate the location and size of imperfect or degraded coating to the component during service inspection.

In embodiments, wherein the metallic electro-mechanical component is a stator metallic component, and the first fluorescent agent is a dye or a pigment; and the outer surface defines a plurality of protrusions and recesses.

The present disclosure provides for an inspection method including providing a metallic electro-mechanical component having an outer surface; and positioning an added layer on the outer surface, the added layer comprising a fluorescent agent; and applying a light source onto the added layer to determine if the added layer was applied uniformly; and re-apply the added layer on the outer surface if the added layer was determined to be not applied uniformly; and positioning a protective coating on the uniformly applied added layer; and applying a light source onto the metallic electro-mechanical component, with the fluorescent agent operating as bright contrast media under the light source to identify imperfect coating of the metallic electro-mechanical component.

In embodiments, wherein by applying light via the light source to the component, areas of spottiness of the protective coating show an indication of reflection from the fluorescent agent of the added layer, in contrast with neighboring protective coated regions of the component with no reflection from the fluorescent agent, and the areas of reflection indicate the location and size of imperfections of the protective coating of the component during a quality control assessment.

In embodiments, the metallic electro-mechanical component is a stator metallic component, and the fluorescent agent is a dye or a pigment; and the outer surface defines a plurality of protrusions and recesses.

The above described and other features are exemplified by the following figures and detailed description.

Any combination or permutation of embodiments is envisioned provided it falls within the scope of the claims. Additional features, functions and applications of the disclosed assemblies, systems and methods of the present disclosure will be apparent from the description which follows, particularly when read in conjunction with the appended figures.

### BRIEF DESCRIPTION OF THE DRAWINGS

The following figures are example embodiments wherein the like elements are numbered alike.

Features and aspects of embodiments are described below with reference to the accompanying drawings, in which elements are not necessarily depicted to scale.

Example embodiments of the present disclosure are further described with reference to the appended figures. It is to be noted that the various features, steps, and combinations of features/steps described below and illustrated in the figures can be arranged and organized differently to result in embodiments which are still within the scope of the present invention as defined by the claims. To assist those of ordinary skill in the art in making and using the disclosed assemblies, systems and methods, reference is made to the appended figures, wherein:
FIG. 1 is a cross-sectional side view of an example coated component, according to the present disclosure;
FIG. 2 is a cross-sectional side view of another example coated component, according to the present disclosure;
FIG. 3 is a cross-sectional side view of another example coated component, according to the present disclosure;
FIG. 4 is a cross-sectional side view of FIG. 1, with a light source for detecting damage to the example coated component;
FIG. 5 is a top view of an example component, after a light source detects damage to the example coated component;
FIG. 6 is a cross-sectional side view of another example coated component, according to the present disclosure; and
FIG. 7 is a cross-sectional side view of another example coated component, according to the present disclosure.

### DETAILED DESCRIPTION

The example embodiments disclosed herein are illustrative of assemblies for quality control and service inspection for metallic electro-mechanical components, and systems of the present disclosure and methods/techniques thereof. It should be understood, however, that the disclosed embodiments are merely examples of the present disclosure, which may be embodied in various forms. Therefore, details disclosed herein with reference to example assemblies for quality control and service inspection for metallic electro-mechanical components and associated processes/techniques of fabrication/assembly and use are not to be interpreted as limiting, but merely as the basis for teaching one skilled in the art how to make and use the assemblies/systems and/or alternative assemblies/systems of the present disclosure.

The present disclosure provides assemblies, systems and methods of quality control and service inspection for metallic electro-mechanical components.

More particularly, the present disclosure provides assemblies, systems and methods of quality control and service inspection for metallic electro-mechanical components (e.g., aircraft metallic stator components) utilizing fluorescent agents.

Current practice provides that a protective coating (e.g., applied sprayed barrier layer) can be used to attempt to protect electro-mechanical components from corrosion; however, quality control of coating coverage can be a challenge for various reasons as discussed above. Moreover, detection of operational corrosion can be needed for pro-active maintenance and/or optimization of a protective coating but accurate detection of such corrosion can be a difficult task.

The present disclosure provides both (i) surface/assembly designs, and (ii) methods of their inspection. In example embodiments of the present disclosure, fluorescent agents can be mixed with protective and/or added layers to serve as bright contrast media under a light source (e.g., ultraviolet ("UV") light source). It is noted that in a case of imperfect coating or coating degraded during service (e.g., due to corrosion), gaps in fluorescent reflection can serve as clear indications of either inadequate quality or excessive corrosion-driven degradation of the components.

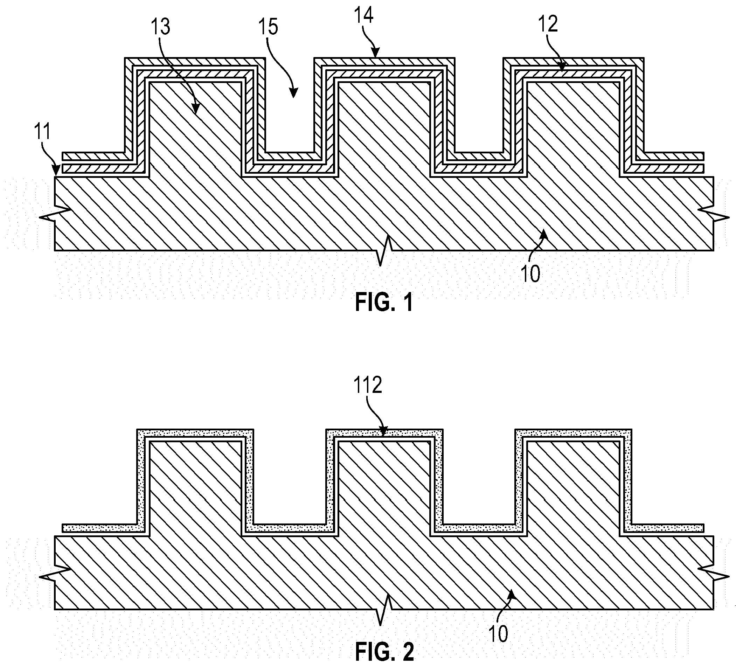

FIG. 1 is a cross-sectional side view of a coated component 10 (e.g., coated metallic electro-mechanical component 10, such as, for example, a coated stator component 10 or the like), according to certain embodiments of the present disclosure.

The component 10 includes a protective coating 12 positioned on a surface 11 (e.g., outer surface 11) of the component 10. It is noted that surface 11 of component 10 can include or define protrusions 13 and/or recesses 15, with protective coating 12 positioned on the protrusions 13 and/or recesses 15 defined by surface 11.

As depicted in FIG. 1, an external added layer 14 can be positioned on the protective coating 12 of component 10. In certain embodiments, the external added layer 14 includes a fluorescent agent, as discussed further below.

FIG. 2 is a cross-sectional side view of another example coated component 10 (e.g., coated metallic electro-mechanical component 10, such as, for example, a coated stator component 10 or the like), according to other embodiments of the present disclosure.

As shown in FIG. 2, component 10 includes a protective coating 112 positioned on a surface 11 of the component 10. In FIG. 2, the protective coating varies from the coating in FIG. 1 at least in that it also includes a fluorescent agent disposed therein.

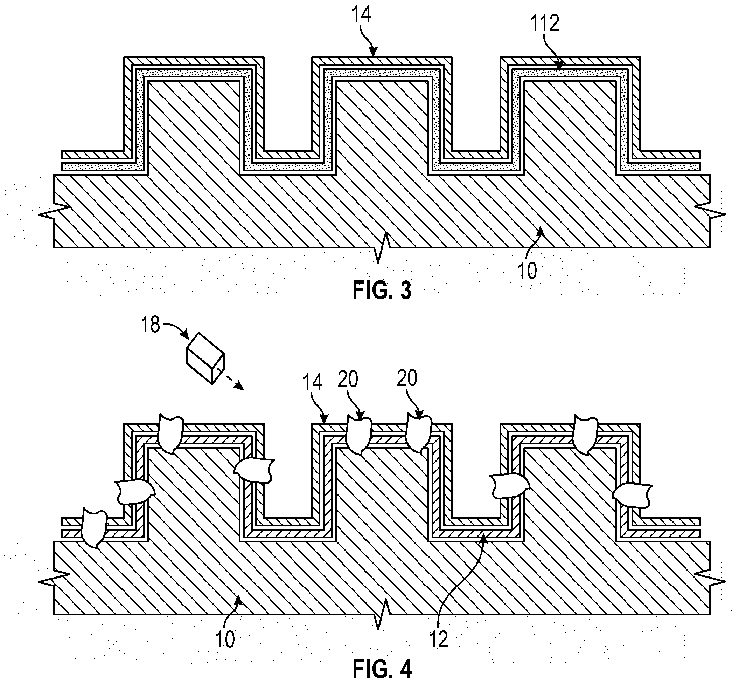

FIG. 3 is a cross-sectional side view of another example coated component 10, according to other embodiments of the present disclosure. As shown in FIG. 3, component 10 includes a protective coating 112 positioned on a surface 11 of the component 10. As depicted in FIG. 3, an external added layer 14 can be positioned on the protective coating 112 of component 10. In certain embodiments, the external added layer 14 includes a first fluorescent agent, and the protective coating 112 includes a second fluorescent agent. In general and without limitation regarding FIG. 3, the second fluorescent agent of coating 112 is different than the first fluorescent agent of layer 14. In such embodiments, the different second fluorescent agent of coating 112 versus the first fluorescent agent of layer 14 provides differences in reflection depending on the type of imperfections of component 10. In other embodiments, it is noted that the first and second fluorescent agents can be the same as one another.

With reference again to FIG. 1, the fluorescent agent of external added layer 14 can be mixed in a polymer matrix of the external added layer 14. As noted and as shown in FIG. 1, this external added layer 14 with the fluorescent agent can be added on top of the existing protective layer 12. In another embodiment and as shown in FIG. 3, this external added layer 14 with the fluorescent agent can be added on top of the existing protective layer 112, with the layer 112 including a fluorescent agent as discussed above. Moreover and with reference to FIG. 2, the protective layer can include a fluorescent agent, and can have no external layer 14 positioned thereon.

In regards to a system and method for service inspection for metallic electro-mechanical components 10 (e.g., a metallic stator component 10 or the like) and with continued reference to FIGS. 1-3, it is noted that the ability of component 10 of FIGS. 1-3 to reflect light in relatively dark confined spaces can be changed due to operational damage (e.g., corrosion, scratches, etc. of layers 12, 112, 14 and/or component 10 during previous use of component 10). By applying light (e.g., UV light) to the component 10 of FIGS. 1-3, the areas of damage of component 10 will show a clear indication of no reflection, in contrast with neighboring undamaged regions of component 10 with bright reflection from the fluorescent agents (from layers 112 and/or 14). These areas of no reflection indicate the location and size of such areas of degraded coating for a more systematic assessment and decision-making process for service inspection for metallic electro-mechanical components 10 (e.g., regarding repair, replacement of component 10 or taking no actions at current level of coating degradation).

FIG. 4 is a cross-sectional side view of FIG. 1, with a light source 18 (e.g., UV light source 18) for detecting surface degradation of the example coated component 10. It is noted that the component 10 of FIGS. 2 and 3 can also be utilized as shown in FIG. 4.

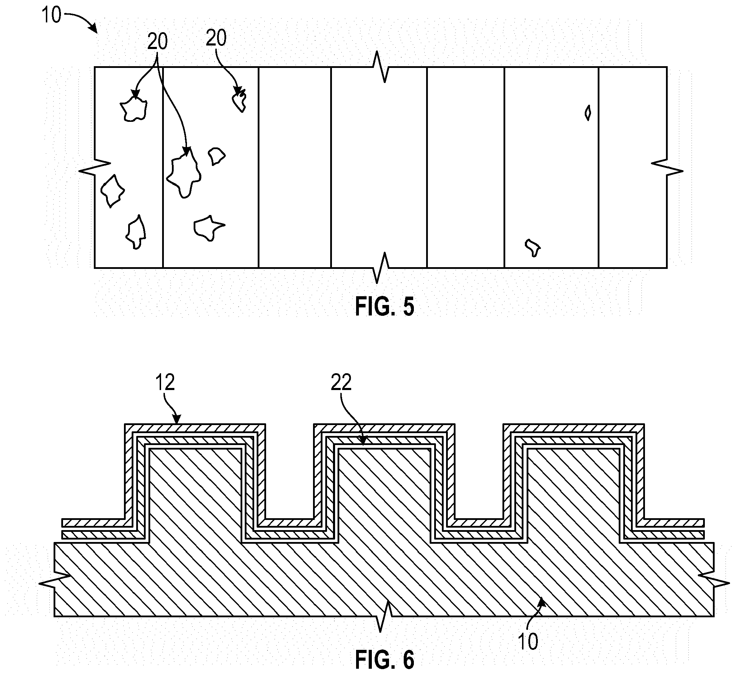

FIG. 5 is a top view of an example component 10, after a light source detects damage to the example coated component 10 (e.g., a surface view of recorded damage patterns to component 10).

As described above and with reference now to FIGS. 4 and 5, by applying light (e.g., UV light) via light source 18 to the component 10 of FIGS. 1-3, the areas of imperfect or degraded (damaged) coating 20 of component 10 will show a clear indication of no reflection 20, in contrast with neighboring undamaged regions of component 10 with bright reflection from the fluorescent agents (from layers 112 and/or 14). These areas of no reflection 20 indicate the location and size of such damages 20 for a more systematic assessment and decision-making process for service inspection for metallic electro-mechanical components 10.

FIG. 6 is a cross-sectional side view of another example coated component 10, according to certain embodiments of the present disclosure. As depicted in FIG. 6, component 10 includes an internal added layer 22 positioned on the surface 11 of component 10. In certain embodiments, the internal added layer 22 includes a fluorescent agent. As shown in FIG. 6, component 10 includes a protective coating 12 positioned on internal added layer 22 of the component 10. In another embodiment, it is noted that coating 112 (with a fluorescent agent) can be positioned on layer 22 instead of coating 12.

With continued reference to FIG. 6, the fluorescent agent of internal added layer 22 can be mixed in a polymer matrix of the internal added layer 22. In example embodiments, the internal added layer 22 is positioned on the metallic surface 11 of component 10 below the coating 12.

In regards to a system and method for quality control for metallic electro-mechanical components 10 (e.g., quality control for new, repaired and/or un-serviced components 10) and with continued reference to FIG. 6, it is noted that the ability of component 10 of FIG. 6 to reflect light in relatively dark confined spaces can be changed due to inadequate coating coverage of coating 12 of component 10 (e.g., spottiness of coating 12). By applying light (e.g., UV light via source 18) to the component 10 of FIG. 6, the areas of spottiness of coating 12 of component 10 will show a clear indication of reflection from layer 22, in contrast with neighboring coated regions 12 of component 10 with no reflection from the fluorescent agent from layer 22. These areas of reflection indicate the location and size of such local imperfections of the coating 12 for a more systematic quality assessment and decision-making process for quality control of metallic electro-mechanical components 10 (e.g., regarding quality-based acceptance/rejection or improvement solutions for component 10).

In example embodiments for a system and method for quality control for components 10, it is noted that in a first step, the component 10 can be covered first with layer 22, the added layer 22 including a fluorescent agent. In a next step, by applying light (e.g., UV light via source 18) to the component 10 with layer 22, a user can check if the reflection of layer 22 is uniform. Layer 22 can be re-applied until it is uniformly applied, via checking with applying light via source 18. After layer 22 is uniformly applied, then coating 12 can be applied to layer 22. After this step, by applying light (e.g., UV light via source 18) to the component 10 with layer 22 and coating 12, a user can check if the there is still reflections of layer 22, with reflection areas of layer 22 indicating missed areas of coating 12. Coating 12 can be re-applied until it is uniformly applied (e.g., via an automated device), via checking with applying light via source 18.

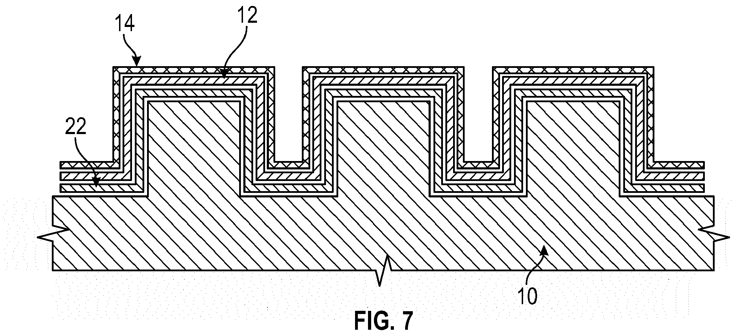

FIG. 7 is a cross-sectional side view of another example coated component 10, according to certain embodiments of the present disclosure. As depicted in FIG. 7, component 10 includes an internal added layer 22 positioned on the surface 11 of component 10. In certain embodiments, the internal added layer 22 includes a fluorescent agent. As shown in FIG. 7, component 10 includes a protective coating 12 positioned on internal added layer 22 of the component 10. In another embodiment, it is noted that coating 112 (with a fluorescent agent) can be positioned on layer 22 instead of coating 12.

With continued reference to FIG. 7, the fluorescent agent of internal added layer 22 can be mixed in a polymer matrix of the internal added layer 22. In example embodiments, the internal added layer 22 is positioned on the metallic surface 11 of component 10 below the coating 12.

As depicted in FIG. 7, an external added layer 14 can be positioned on the protective coating 12 of component 10. In certain embodiments, the external added layer 14 includes a fluorescent agent (e.g., different than the fluorescent agent of layer 22, although the present disclosure is not limited thereto).

In a system and method for quality control for components 10 (e.g., quality control for new, repaired and/or un-serviced components 10) and with continued reference to FIG. 7, it is noted that the ability of un-serviced component 10 of FIG. 7 to reflect light in relatively dark confined spaces can be changed due to inadequate coating coverage of coating 12 of component 10 (e.g., spottiness of coating 12, before applying layer 14). By applying light (e.g., UV light via source 18) to the component 10 of FIG. 7 (before applying layer 14), the areas of spottiness of coating 12 of component 10 will show a clear indication of reflection from layer 22, in contrast with neighboring coated regions 12 of component 10 with no reflection from the fluorescent agent from internal layer 22. These areas of reflection indicate the location and size of such local imperfections of the coating 12 for a more systematic quality assessment and decision-making process for quality control of metallic electro-mechanical components 10 (e.g., regarding acceptance/rejection or improvement solutions for component 10). As such, the present disclosure provides the coatings/layers can be applied as a two-step process, e.g., checking quality before applying layer 14 and then after applying layer 14, the process can be used for assessment of degradations during service, as described below.

With continued reference to FIG. 7 and in regards to a system and method for service inspection for components 10, it is noted that the ability of component 10 of FIG. 7 to reflect light in relatively dark confined spaces can be changed due to service damage (e.g., corrosion, scratches, etc. of layers 12, 112, 14, 22 and/or component 10 during previous use of component 10). By applying light (e.g., UV light via source 18) to the component 10 of FIG. 7, the areas of damage of component 10 will show a clear indication of no reflection, in contrast with neighboring undamaged regions of component 10 with bright reflection from the fluorescent agents (from layers 112, 14 and/or 22). These areas of no reflection indicate the location and size of such damages 20 for a more systematic assessment and decision-making process for service inspection for metallic electro-mechanical components 10 (e.g., regarding repair, replacement of component 10).

Some example fluorescent agents for layers 14, 22 and/or coating 112 include, without limitation, dyes and/or pigments.

For example, fluorescent agents and/or dyes that absorb UV light (e.g., around 350 nm) and fluoresce in the visible spectrum can be utilized (e.g., added in the polymer composite resins and/or fibers of layers 14, 22 and/or coating 112).

Some example fluorescent agents include, without limitation: anthrathioxanthene, thioxanthene benzanthrone or anthraquinone series based chemistries. One example has the following molecular formula:

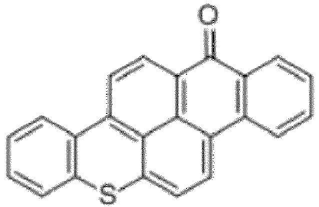

These family of colorants are known under the trade names Solvent Orange 63, Fluorescent Red GG, Hostasol Red GG, or Macolox Fluorescent Red GG.

In other example embodiments, xanthene, benzothioxanthene-dicarboximide, and aminoketones series-based chemistries can be utilized. Examples have the following molecular formulae:

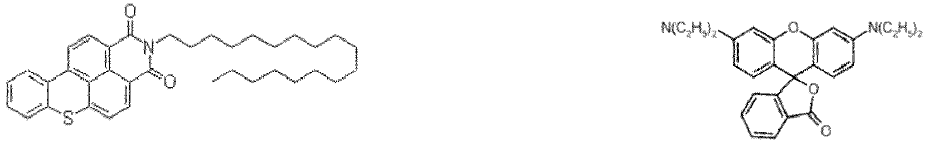

These family of colorant(s) are known under the trade names Solvent yellow 98, Fluorescent YELLOW 3GF, Hostasol Yellow 3G, Solvent Fluorescent Yellow 3G, Rosaplast Yellow F5G, Keyplast Fluorecent Yellow 3R, RADGLo CFS-6-03 RED or Solvent Red 49.

Other examples include coumarin, coumarin 480, hydroxycoumarin, and glycidyl-oxycoumarin of the molecular formulae below:

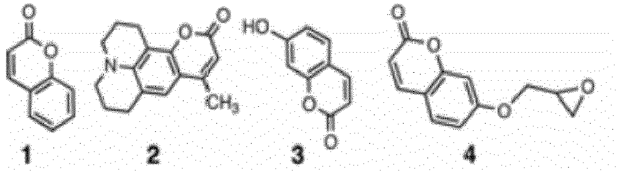

These chemistries upon UV excitation fluoresce in the blue range of the visible spectrum (e.g., 400 to 500 nm).

Another example includes benzopyran based chemistry. One example has the following molecular formula:

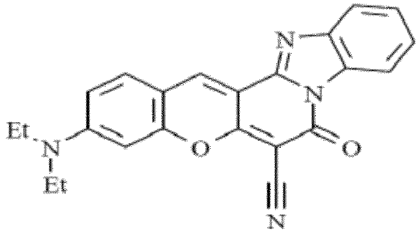

This colorant is known under the trade name RADGLO CFS-6-02 RED, and Solvent Red 196.

Another example is azomethine based chemistry. One example has the following molecular formula:

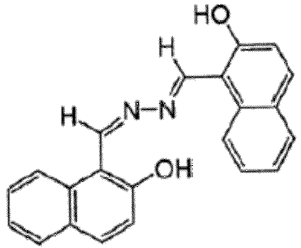

This colorant is known under the trade name RADGLO VSF-0-01 Yellow. Upon UV excitation, it fluoresces in yellow/green color.

Other examples include naphthalimide and perylene based chemistries. Examples have the following molecular formulae:

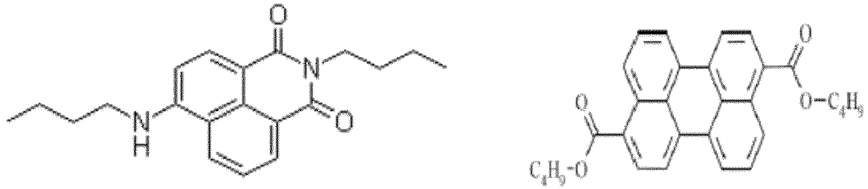

These colorants are known under the trade name RADGLO CFS-0-01 Yellow or Solvent Yellow 43, RADGLO CFS-0-05 or Solvent Green 5. Upon UV excitation, it fluoresces in yellow/green color.

Some various UV fluorescent thermoset resin pigments under several commercial names can be utilized, such as, for example: (i) RADGLO^{®} P-09 UV Blue pigment (solid solution of dyestuffs in a thermoset sulphonamide-melamine-paraformaldehyde polymer resin); (ii) RADGLO GM family of dyed/pigmented (range of colors) thermoset modified benzoguanamine-formaldehyde copolymer; (iii) RADGLO GRT family of dyed/pigmented (range of colors) thermoset polyester amide copolymers; and/or (iv) RADGLO PC and RADGLO PS families of dyed/pigmented (range of colors) thermoset sulphonamide-melamine-paraformaldehyde polymer resin.

There are many benefits of the assemblies, systems and methods of the present disclosure, including, without limitation: high reliability of detection of inadequate quality or excessive corrosion degradation; efficiency (convenience and robustness) of implementation; low cost and low demand of specially trained labor; opportunity to apply it as express method for coating optimization and/or benchmarking; opportunity to use available fluorescent agents; and/or the opportunity to utilize with both aircraft and non-aircraft components.

While particular embodiments have been described, alternatives, modifications, variations, improvements, and substantial equivalents that are or may be presently unforeseen may arise to applicants or others skilled in the art. Accordingly, the appended claims as filed and as they may be amended are intended to embrace all such alternatives, modifications variations, improvements, and substantial equivalents.

The ranges disclosed herein are inclusive of the endpoints, and the endpoints are independently combinable with each other (e.g., ranges of "up to 25 wt.%, or, more specifically, 5 wt.% to 20 wt.%", is inclusive of the endpoints and all intermediate values of the ranges of "5 wt.% to 25 wt.%," etc.). "Combinations" is inclusive of blends, mixtures, alloys, reaction products, and the like. The terms "first," "second," and the like, do not denote any order, quantity, or importance, but rather are used to distinguish one element from another. The terms "a" and "an" and "the" do not denote a limitation of quantity and are to be construed to cover both the singular and the plural, unless otherwise indicated herein or clearly contradicted by context. "Or" means "and/or" unless clearly stated otherwise. Reference throughout the specification to "some embodiments", "an embodiment", and so forth, means that a particular element described in connection with the embodiment is included in at least one embodiment described herein, and may or may not be present in other embodiments. In addition, it is to be understood that the described elements may be combined in any suitable manner in the various embodiments. A "combination thereof' is open and includes any combination comprising at least one of the listed components or properties optionally together with a like or equivalent component or property not listed.

Unless defined otherwise, technical and scientific terms used herein have the same meaning as is commonly understood by one of skill in the art to which this application belongs.

Although the systems and methods of the present disclosure have been described with reference to example embodiments thereof, the present disclosure is not limited to such example embodiments and/or implementations. Rather, the systems and methods of the present disclosure are susceptible to many implementations and applications, as will be readily apparent to persons skilled in the art from the disclosure hereof, and as fall within the scope of the claims. The present disclosure expressly encompasses such modifications, enhancements and/or variations of the disclosed embodiments. Since many changes could be made in the above construction and many widely different embodiments of this disclosure could be made without departing from the scope of the claims, it is intended that all matter contained in the drawings and specification shall be interpreted as illustrative and not in a limiting sense. Additional modifications, changes, and substitutions are intended in the foregoing disclosure.

## Claims

1. A coated assembly comprising:
a metallic electro-mechanical component (10) having an outer surface (11); and
a protective coating (12) or a first added layer (14) positioned proximal to the outer surface, the protective coating or the first added layer comprising a first fluorescent agent;
wherein the first fluorescent agent operates as bright contrast media under a light source to identify imperfect coating or coating degradation during service of the metallic electro-mechanical component.

2. The assembly of claim 1, wherein the protective coating is positioned on the outer surface, and the first added layer is positioned on the protective coating

3. The assembly of claim 2, wherein the first added layer comprises the first fluorescent agent, and the protective coating is free of the first fluorescent agent.

4. The assembly of claim 1, wherein the protective coating is positioned on the outer surface, and the metallic electro-mechanical component is free of the first added layer, the protective coating comprising the first fluorescent agent.

5. The assembly of claim 2, wherein the protective coating comprises the first fluorescent agent, and the first added layer comprises a second fluorescent agent.

6. The assembly of claim 1, wherein the first added layer is positioned on the outer surface, and the protective coating is positioned on the first added layer, the first added layer comprising the first fluorescent agent.

7. The assembly of claim 6, wherein the protective coating comprises a second fluorescent agent.

8. The assembly of claim 6 further comprising a second added layer positioned on the protective coating, the second added layer comprising a second fluorescent agent, and optionally wherein the protective coating comprises a third fluorescent agent.

9. The assembly of any preceding claim, wherein the metallic electro-mechanical component is a stator metallic component, and the first fluorescent agent is a dye or a pigment; and
wherein the outer surface defines a plurality of protrusions and recesses.

10. An inspection method comprising:
providing a metallic electro-mechanical component (10) having an outer surface (11); and
positioning a protective coating (12) or a first added layer (14) proximal to the outer surface, the protective coating or the first added layer comprising a first fluorescent agent;
applying a light source (18) onto the metallic electro-mechanical component, with the first fluorescent agent operating as bright contrast media under the light source to identify imperfect coating or coating degradation during service of the metallic electro-mechanical component.

11. The method of claim 10, wherein the protective coating is positioned on the outer surface, and the first added layer is positioned on the protective coating; and
wherein the first added layer comprises the first fluorescent agent, and the protective coating is free of the first fluorescent agent; and
wherein by applying light via the light source to the component, areas of imperfect or degraded coating of the component show an indication of no reflection of light, in contrast with neighboring undamaged regions of the component having bright reflection from the first fluorescent agent, and the areas of no reflection indicate the location and size of imperfect or degraded coating to the component during service inspection; or
wherein the protective coating is positioned on the outer surface, and the metallic electro-mechanical component is free of the first added layer, the protective coating comprising the first fluorescent agent; and
wherein by applying light via the light source to the component, areas of imperfect or degraded coating of the component show an indication of no reflection of light, in contrast with neighboring undamaged regions of the component having bright reflection from the first fluorescent agent, and the areas of no reflection indicate the location and size of imperfect or degraded coating to the component during service inspection; or
wherein the protective coating is positioned on the outer surface, and the first added layer is positioned on the protective coating; and
wherein the protective coating comprises the first fluorescent agent, and the first added layer comprises a second fluorescent agent;
wherein by applying light via the light source to the component, areas of imperfect or degraded coating of the component show an indication of no reflection of light, in contrast with neighboring undamaged regions of the component having bright reflection from the first or second fluorescent agents, and the areas of no reflection indicate the location and size of imperfect or degraded coating to the component during service inspection; or
wherein the first added layer is positioned on the outer surface, and the protective coating is positioned on the first added layer, the first added layer comprising the first fluorescent agent; and
wherein by applying light via the light source to the component, areas of spottiness of the protective coating show an indication of reflection from the first fluorescent agent of the first added layer, in contrast with neighboring protective coated regions of the component with no reflection from the first fluorescent agent, and the areas of reflection indicate the location and size of imperfections of the protective coating of the component during a quality control assessment; or
wherein the first added layer is positioned on the outer surface, and the protective coating is positioned on the first added layer, the first added layer comprising the first fluorescent agent;
further comprising a second added layer positioned on the protective coating, the second added layer comprising a second fluorescent agent;
wherein by applying light via the light source to the component, areas of imperfect or degraded coating of the component show an indication of no reflection of light, in contrast with neighboring undamaged regions of the component having bright reflection from the first or second fluorescent agents, and the areas of no reflection indicate the location and size of imperfect or degraded coating to the component during service inspection.

12. The method of claim 10, wherein the metallic electro-mechanical component is a stator metallic component, and the first fluorescent agent is a dye or a pigment; and
wherein the outer surface defines a plurality of protrusions and recesses.

13. An inspection method comprising:
providing a metallic electro-mechanical component (10) having an outer surface (11); and
positioning an added layer (14) on the outer surface, the added layer comprising a fluorescent agent;
applying a light source (18) onto the added layer to determine if the added layer was applied uniformly;
re-apply the added layer on the outer surface if the added layer was determined to be not applied uniformly;
positioning a protective coating on the uniformly applied added layer;
applying a light source onto the metallic electro-mechanical component, with the fluorescent agent operating as bright contrast media under the light source to identify imperfect coating of the metallic electro-mechanical component.

14. The method of claim 13, wherein by applying light via the light source to the component, areas of spottiness of the protective coating show an indication of reflection from the fluorescent agent of the added layer, in contrast with neighboring protective coated regions of the component with no reflection from the fluorescent agent, and the areas of reflection indicate the location and size of imperfections of the protective coating of the component during a quality control assessment.

15. The method of claim 13 or 14, wherein the metallic electro-mechanical component is a stator metallic component, and the fluorescent agent is a dye or a pigment; and
wherein the outer surface defines a plurality of protrusions and recesses.
